# EUROPÄISCHE PATENTSCHRIFT

(11) **EP 0 591 795 B1**
(45) Veröffentlichungstag und Bekanntmachung des Hinweises auf die Patenterteilung: **31.01.1996**
(21) Anmeldenummer: 93115507.1
(22) Anmeldetag: 25.09.1993
(51) Int. Cl.: C07D 307/06, C07D 307/12, C07D 307/16, C07D 315/00, C07D 309/06, C07C 29/149, C07C 55/22, C07C 67/327

(54) **Verfahren zur Hydrierung von Zitronensäure**
Process for hydrogenolysis of citric acid
Procédé d'hydrogénolyse d'acide citrique

(30) Priorität: 05.10.1992 DE 4233431
(43) Veröffentlichungstag der Anmeldung: 13.04.1994
(73) Patentinhaber: BASF Aktiengesellschaft, D-67063 Ludwigshafen (DE)
(72) Erfinder: Weyer, Hans-Juergen, Dr., D-6800 Mannheim 1 (DE); Fischer, Rolf, Dr., D-6900 Heidelberg (DE); Sigwart, Christoph, Dr., D-6905 Schriesheim (DE)

(56) Entgegenhaltungen:
- EP-A- 0 044 444
- EP-A- 0 277 562
- EP-A- 0 394 842
- WO-A-82/03854
- HOUBEN-WEYL 'Band 4/1c' 1980 , THIEME VERLAG , STUTTGART DE

## Beschreibung

Die vorliegende Erfindung betrifft ein neues Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 13-(2'-Hydroxyethyl)tetrahydrofuran, 4-Hydroxymethyltetrahydropyran, 2-Methyl-γ-butyrolacton und/oder 3-Methyl-γ-butyrolacton durch katalytische Hydrierung von Zitronensäure und/oder deren C₁ - bis C₂₀-Alkyl- oder C₇- bis C₁₂-Aralkylestern in nicht-wäßrigen Lösungsmitteln.

Aus der EP-A-277 562 ist die katalytische Hydrierung von Zitronensäure zu difunktionelle Verbindungen wie 3-Methyltetrahydrofuran und 3- bzw. 4-Methylbutyrolacton bekannt, doch werden nach diesem speziellen Verfahren keine trifunktionellen Verbindungen erhalten.

Es bestand daher die Aufgabe, ein Verfahren zu entwickeln, das den vorgenannten Nachteilen abhilft.

Demgemäß wurde ein neues und verbessertes Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3- (2'-Hydroxyethyl)tetrahydrofuran, 4-Hydroxymethyltetrahydropyran, 2-Methyl-γ-butyrolacton und/oder 3-Methyl-γ-butyrolacton gefunden, welches dadurch gekennzeichnet ist, daß man Zitronensäure oder deren C₁- bis C20-Alkyl- oder C₇- bis C₁₂-Aralkylester in nicht-wäßrigen Lösungsmitteln bei Temperaturen von 50 bis 400°C und Drücken von 1 bis 400 bar an Hydrierkatalysatoren umsetzt.

Das erfindungsgemäße Verfahren läßt sich wie folgt durchführen:

Zitronensäure und/oder deren C₁- bis C₂₀-Alkyl- oder C₇- bis C₁₂-Aralkylester lassen sich bei Temperaturen von 50 bis 400°C, bevorzugt bei 150 bis 300°C und Drücken von 1 bis 400 bar, bevorzugt 50 bis 300 bar katalytisch an Hydrierkatalysatoren in nicht-wäßrigen Lösungsmitteln umsetzen.

Als Zitronensäureester eignen sich beispielsweise Monoester wie Zitronensäuremonomethylester, Zitronensäuremonoethylester, Zitronensäuremono-n-propylester, Zitronensäuremono-iso-propylester, Zitronensäuremono-n-butylester, Zitronensäuremono-iso-butylester,

Zitronensäuremono-sec.-butylester und Zitronensäuremono-tert.-butylester, Diester wie Zitronensäuredimethylester, Zitronensäurediethylester, Zitronensäuredi-n-propylester, Zitronensäuredi-iso-propylester, Zitronensäuredi-n-butylester, Zitronensäuredi-iso-butylester, Zitronensäuredi-sec.-butylester und Zitronensäuredi-tert.-butylester, und Triester wie Zitronensäuretrimethylester, Zitronensäuretriethylester, Zitronensäuretri-n-propylester, Zitronensäuretri-iso-propylester, Zitronensäuretri-n-butylester, Zitronensäuretri-iso-butylester, Zitronensäuretri-sec.-butylester, Zitronensäuretri-tert.-butylester und Zitronensäuretribenzylester sowie deren Gemische. Es können auch alle bei der erfindungsgemäßen Hydrierung als Zwischenstufe durchlaufenen Verbindungen eingesetzt werden. Alle diese Einsatzstoffe können in fester, flüssiger oder gasförmiger Form in das erfindungsgemäße Verfahren eingebracht werden. Besonders bevorzugt ist die Verwendung von Zitronensäure und Zitronensäuretrialkylestern wie Zitronensäuretrialkylester mit C₁- bis C₂₀-Alkylgruppen, bevorzugt Zitronensäuretrialkylester mit C₁- bis C₈-Alkylgruppen z.B. Zitronensäuretrimethylester, Zitronensäuretriethylester, Zitronensäuretri-n-propylester, Zitronensäuretri-iso-propylester, Zitronensäuretri-n-butylester, Zitronensäuretri-iso-butylester, Zitronensäuretri-sec.-butylester, Zitronensäuretri-tert.-butylester, Zitronensäuretri-n-hexylester und Zitronensäuretriamylester, besonders bevorzugt Zitronensäuretrialkylester mit C₁- bis C₄-Alkylgruppen z.B. Zitronensäuretrimethylester, Zitronensäuretriethylester, Zitronensäuretri-n-propylester, Zitronensäuretri-iso-propylester, Zitronensäuretri-n-butylester, Zitronensäuretri-iso-butylester, Zitronensäuretri-sec.-butylester und Zitronensäuretri-tert.-butylester.

Die Hydrierung kann in nicht-wäßrigen Lösungsmitteln durchgeführt werden, wobei die Zitronensäure und/oder deren Ester bzw. deren Reaktionsprodukte ebenfalls als Lösungsmittel fungieren können; ausgenommen sind wäßrige Reaktionssysteme.

Nicht-wäßrige Lösungsmittel sind solche, denen kein Wasser zugefügt wurde bzw. wird. Die nicht-wäßrigen Lösungsmittel können feucht sein, d.h. sie bedürfen nicht der Trocknung, also der Entfernung von Restwasser (Hygroskopie). Als nicht-wäßrige Lösungsmittel eignen sich beispielsweise Ether wie Dialkylether, bevorzugt Dialkylether mit C₁- bis C₂₀-Alkylgruppen, besonders bevorzugt Dialkylether mit C₁- bis C₈-Alkylgruppen, z.B. Diethylether, Methyl-tert.-butylether, Di-n-butylether, Ethylenglykoldimethylether, Ethylenglykoldiethylether und Diglyme und cyclische Ether wie 5- bis 8-Ringether z.B. Furan, Tetrahydrofuran, Pyran, Dihydropyran und Dioxan, bevorzugt Ethylenglykoldimethylether, Ethylenglykoldiethylether, Tetrahydrofuran und Dioxan, besonders bevorzugt Ethylenglykoldiethylether, Tetrahydrofuran, Alkohole, die z.B. 70 bis 100 %ig sind, bevorzugt C₁- bis C₆-Alkanole wie Methanol, Ethanol, n-Propanol, iso-Propanol, n-Butanol, iso-Butanol, sec.-Butanol, tert.-Butanol, n-Pentanol und n-Hexanol, besonders bevorzugt C₁- bis C₄-Alkanole wie Methanol, Ethanol, n-Propanol, iso-Propanol und n-Butanol. Es können auch beliebige Gemische der vorgenannten Lösungsmittel eingesetzt werden.

Als Hydrierkatalysatoren eignen sich alle übliche Katalysatoren wie sie beispielweise in Houben-Weyl, Methoden der organischen Chemie, Band IV/1 c, Georg Thieme Verlag Stuttgart, 1980, beschrieben sind. Bevorzugte Hydrierkatalysatoren sind solche, deren katalytisch aktive Masse eines oder mehrere Metalle der I., VII. oder VIII. Nebengruppe des Periodensystems der Elemente wie Kupfer, Silber, Gold, Mangan, Rhenium, Eisen, Cobalt, Nickel, Ruthenium, Rhodium, Palladium, Osmium, Iridium und Platin, bevorzugt Kupfer, Eisen, Nickel, Cobalt, Palladium, Platin, Rhodium und Ruthenium, besonders bevorzugt Kupfer, Palladium, Ruthenium, Nickel und Cobalt, sowie gegebenenfalls eines oder mehrere Metalle der II. bis VI. Nebengruppe des Periodischen Systems der Elemente wie Zink, Cadmium, Quecksilber, Scandium, Yttrium, Lanthan, Titan, Zirkon, Hafnium, Vanadin, Niob, Tantal, Chrom, Molybdän und Wolfram, bevorzugt Zink, Chrom, Molybdän, Lanthan, Zirkon und Wolfram, besonders bevorzugt Zink, Chrom, Molybdän und Wolfram, sowie gegebenenfalls Elemente der I. und II. Hauptgruppe des Perioden- systems der Elemente wie Lithium, Natrium, Kalium, Rubidium, Cäsium, Francium, Magnesium, Calcium, Strontium, Barium und Radium, bevorzugt Lithium, Natrium, Kalium, Magnesium und Calcium enthalten.

Diese Katalysatoren liegen im allgemeinen bei der Hydrierung in Form ihrer Oxide vor und können zusätzlich Säuren enthalten wie Phosphorsäure, Schwefelsäure, Flußwasserstoffsäure und Heteropolysäuren, bevorzugt Phosphorsäure und Heteropolysäuren, besonders bevorzugt Phosphorsäure.

Die Hydrierkatalysatoren können als Homogen- oder bevorzugt als Heterogenkatalysatoren eingesetzt werden. Werden Heterogenkatalysatoren verwendet, können sie sowohl als Trägerkatalysatoren oder in kompakter Form eingesetzt werden. Die Art des Trägermaterials ist in der Regel nicht kritisch, es können übliche Trägermaterialien wie Siliciumdioxid, Aluminiumoxide, Titandioxid, Zirkondioxid, Aktivkohle, Silikate oder Zeolithe verwendet werden. Erforderlichenfalls können zur Herstellung der Katalysatoren Bindemittel oder Formhilfsmittel mitverwendet werden.

Besonders Edelmetallkatalysatoren werden üblicherweise auf Trägern wie Kohle (z.B. Aktivkohle), Aluminiumoxid oder Zirkondioxid eingesetzt, beispielsweise Palladium auf Kohle, Palladium auf Aluminiumoxid, Platin auf Kohle, Ruthenium auf Kohle und Ruthenium auf Zirkondioxid.

Die Hydrierung kann in der Gas- oder Flüssigphase durchgeführt werden. Man kann sowohl diskontinuierlich als auch bevorzugt kontinuierlich arbeiten. Wird ein Heterogenkatalysator verwendet, kann dieser als Suspensions- oder Festbettkatalysator eingesetzt werden. Als Reaktoren können Rührkessel oder Rohrreaktoren eingesetzt werden. Ein Rohrreaktor mit fest angeordnetem Katalysator kann in der Sumpffahrweise oder der Rieselfahrweise betrieben werden. Dabei haben sich Katalysatorbelastungen von 0,01 bis 2, insbesondere von 0,05 bis 0,5 kg Zitronensäure bzw. deren Derivate je Liter Katalysator und Stunde bewährt.

Je nach Reaktionsbedingungen, verwendetem Lösungsmittel und Katalysator lassen sich bevorzugt Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/oder 3-Methyl-γ-butyrolacton herstellen:

Dabei ist im allgemeinen die Bildung der trifunktionellen Verbindungen Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-(2'-Hydroxyethyl)-tetrahydrofuran und 4-Hydroxymethyltetrahydropyran dann bevorzugt, wenn in alkoholischen Lösungsmitteln gearbeitet wird und/oder Zitronensäureester eingesetzt werden.

Dagegen entstehen die difunktionellen Verbindungen 2-Methyl-γ-butyrolacton, 3-Methyl-γ-butyrolacton und 3-Methyltetrahydrofuran bevorzugt, wenn Zitronensäure in etherischen Lösungsmitteln eingesetzt werden.

Im allgemeinen verhält es sich so, daß durch niedrige Reaktionstemperaturen und -drücke sowie kurze Verweilzeiten die Bildung von Propan-1,2,3-tricarbonsäure und deren Ester bevorzugt wird, da zuerst die tertiäre Hydroxylgruppe abgespalten wird. Demgegenüber fördern hohe Reaktionstemperaturen und -drücke sowie lange Verweilzeiten die Bildung der Ether 3-(2'-Hydroxyethyl)-tetrahydrofuran, 4-Hydroxymethyltetrahydropyran und 3-Methyltetrahydrofuran. Tetrahydrofurylessigsäure und deren Ester entstehen im allgemeinen bevorzugt bei mittleren Reaktionstemperaturen, -drücken und Verweilzeiten. Für die Hydrierung zu Propan-1,2,3-trimethanol sind niedrige Reaktionstemperaturen, hohe Reaktionsdrücke sowie kurze Verweilzeiten günstig. 2-Methyl-γ-butyrolacton und 3-Methyl-γ-butyrolacton werden bevorzugt bei mittleren Reaktionstemperaturen und -drücken sowie kurzen Verweilzeiten gebildet.

So entsteht Propan-1,2,3-tricarbonsäure und deren Ester im allgemeinen bevorzugt bei Temperaturen von 100 bis 175°C, insbesondere von 125 bis 175°C und Drücken von 1 bis 200 bar, insbesondere von 10 bis 100 bar.

Tetrahydrofurylessigsäure und deren Ester entstehen bevorzugt bei Temperaturen von 125 bis 250°C, insbesondere von 150 bis 200°C und Drücken von 1 bis 200 bar, insbesondere von 50 bis 150 bar.

Propan-1,2,3-trimethanol entsteht bevorzugt bei Temperaturen von 100 bis 250°C, insbesondere von 125 bis 175°C und Drücken von 100 bis 400 bar, insbesondere von 150 bis 300 bar.

2-Methyl-γ-butyrolacton und 3-Methyl γ-butyrolacton entstehen bevorzugt bei Temperaturen von 100 bis 200°C, insbesondere von 125 bis 175°C und Drücken von 50 bis 400 bar, insbesondere von 100 bis 300 bar.

3-(2'-Hydroxyethyl)-tetrahydrofuran, 4-Hydroxymethyltetrahydropyran sowie 3-Methyltetrahydrofuran entstehen bevorzugt bei Temperaturen von 150 bis 300°C, insbesondere von 175 bis 275°C und Drücken von 100 bis 400 bar, insbesondere von 200 bis 300 bar.

Die jeweiligen Reaktionsbedingungen, unter denen die einzelnen Produkte bevorzugt gebildet werden, hängen stark von den verwendeten Katalysatoren ab.

Die Abspaltung der tertiären Hydroxylgruppe sowie die Tetrahydrofuran- bzw. Tetrahydropyran-Bildung werden durch die Anwesenheit von sauer wirkenden Substanzen verstärkt.

Die nach dem erfindungsgemäßen Verfahren herstellbaren Produkte gehören zu den Gruppen der Tricarbonsäuren, Triole und Etherole und dienen z.B. bei der Herstellung beispielsweise von Polyethern (Römpps Chemie-Lexikon, 8. Auflage, S. 3287, Stuttgart, 1987; Houben-Weyl, Bd. 14/2, Stuttgart, 1963, S. 580; Ullmann's Encyklopädie der technischen Chemie, 3. Auflage, München, 1963, S. 43), Polyester (Ullmann's Encyklopädie der technischen Chemie, München, 1963, Bd. 14, S. 80; Römpps Chemie-Lexikon, Stuttgart, 1987, S. 3285; Houben-Weyl, Bd. 14/2, Stuttgart, 1963, S. 1) und Polyurethanen (Römpp, S. 3318; Houben-Weyl, Bd. 14/2, S. 57; Ullmann, Bd. 14, S. 338) als Comonomere.

### Beispiele

Die in den Beispielen eingesetzten Katalysatoren haben im nicht-reduzierten Zustand folgende Zusammensetzung:

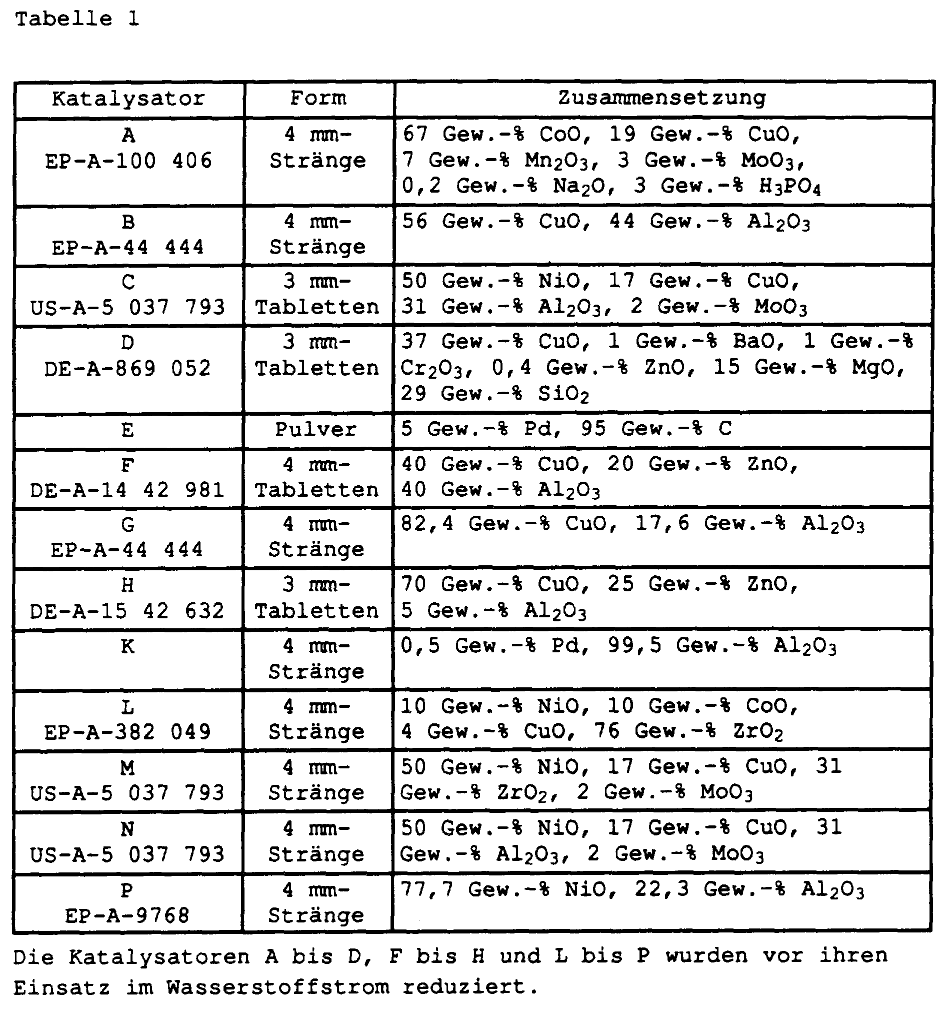

Die nachstehenden Ausbeuten wurden gaschrmatographisch ermittelt.

### Beispiel 1

400 ml Zitronensäuretriethylester wurden zusammen mit 1100 ml Tetrahydrofuran und 60 g des Katalysators C (3 mm-Tabletten) bei 225°C und 200 bar solange hydriert, bis keine Wasserstoffaufnahme mehr zu beobachten war. Der Reaktionsaustrag wurde von Tetrahydrofuran und Ethanol befreit und im Vakuum destilliert. Dabei wurden als Hauptprodukt 118 g (61 %) 3-(2'-Hydroxyethyl)tetrahydrofuran erhalten. Weiterhin waren 4 g (2%) 4-Hydroxymethyltetrahydropyran entstanden.

### Beispiel 2

400 g Zitronensäure wurden in 1000 ml n-Butanol gelöst und zusammen mit 60 g des Katalysators A (4 mm-Stränge) bei 250°C und 200 bar solange hydriert, bis keine Wasserstoffaufnahme mehr zu beobachten war. Der Reaktionsaustrag wurde von n-Butanol und Ethanol befreit und im Vakuum destilliert. Es wurden 99 g (52 %) 3-(2'-Hydroxyethyl)tetrahydrofuran und 2,9 g (1,5%) 4-Hydroxy-methyltetrahydropyran erhalten.

### Beispiel 3

400 ml Zitronensäuretriethylester wurden zusammen mit 1100 ml Ethanol und 60 g des Katalysators B (4 mm-Stränge) bei 175°C und 10 bar solange hydriert, bis keine Wasserstoffaufnahme mehr zu beobachten war. Der Reaktionsaustrag wurde vom Ethanol befreit und im Vakuum destilliert. Es wurden 231,8 g (54 %) 1,2,3-Propantricarbonsäuretriethylester erhalten.

### Beispiel 4

400 ml Zitronensäuretriethylester wurden zusammen mit 1100 ml Ethanol und 60 g des Katalysators B (4 mm-Stränge) bei 200°C und 200 bar solange hydriert, bis keine Wasserstoffaufnahme mehr zu beobachten war. Der Reaktionsaustrag wurde vom Ethanol befreit und im Vakuum destilliert. Es wurden 80 g (42 %) 3-(2'-Hydroxyethyl)tetrahydrofuran und 1,8 g (1 %) 4-Hydroxymethyltetrahydropyran erhalten.

### Beispiel 5

400 ml Zitronensäuretriethylester wurden zusammen mit 1100 ml Tetrahydrofuran und 60 g des Katalysators H (3 mm-Tabletten) bei 225°C und 200 bar solange hydriert, bis keine Wasserstoffaufnahme mehr zu beobachten war. Der Reaktionsaustrag wurde von Tetrahydrofuran und Ethanol befreit und im Vakuum destilliert. Es wurden 93,6 g (49 %) 3-(2'-Hydroxyethyl)tetrahydrofuran und 6,8 g (3,6 %) 4-Hydroxymethyltetrahydropyran erhalten.

### Beispiel 6

400 ml Zitronensäuretriethylester wurden zusammen mit 1100 ml Ethanol und 10 g des Katalysators E (Pulver) bei 200°C und 50 bar solange hydriert, bis keine Wasserstoffaufnahme mehr zu beobachten war. Der Reaktionsaustrag wurde vom Ethanol befreit und im Vakuum destilliert. Es wurden 166,1 g (38 %) Propantricarbonsäuretriethylester erhalten.

### Beispiel 7

400 ml Zitronensäuretriethylester wurden zusammen mit 1100 ml Tetrahydrofuran und 60 g des Katalysators D (3 mm-Tabletten) bei 150°C und 200 bar solange hydriert, bis keine Wasserstoffaufnahme mehr zu beobachten war. Der Reaktionsaustrag wurde vom Tetrahydrofuran befreit und im Vakuum destilliert. Es wurden 87 g (39 %) Propan-1,2,3-trimethanol und 41,6 g (19 %) 3-(2'-Hydroxyethyl)tetrahydrofuran erhalten.

### Beispiel 8

400 ml Zitronensäuretriethylester wurden zusammen mit 1100 ml Tetrahydrofuran und 60 g des Katalysators F (4 mm-Tabletten) bei 175°C und 50 bar solange hydriert, bis keine Wasserstoffaufnahme mehr zu beobachten war. Der Reaktionsaustrag wurde vom Tetrahydrofuran befreit und im Vakuum destilliert. Es wurden 101 g (39 %) Tetrahydrofurylessigsäureethylester und 57 g (30 %) 3-(2'-Hydroxyethyl)tetrahydrofuran erhalten.

### Beispiel 9

400 ml Zitronensäuretriethylester wurden zusammen mit 1100 ml Tetrahydrofuran und 60 g des Katalysators G (4 mm-Tabletten) bei 175°C und 10 bar solange hydriert, bis keine Wasserstoffaufnahme mehr zu beobachten war. Der Reaktionsaustrag wurde vom Ethanol befreit und im Vakuum destilliert. Es wurden 193 g (45 %) Propan-1,2,3-tricarbonsäuretriethylester erhalten.

### Beispiele 10 bis 16

70 g Zitronensäure wurden zusammen mit 1000 ml Ethylenglykoldimethylether und 50 ml Katalysators bei 200 bar und der in der Tabelle 2 angegebenen Hydriertemperatur solange hydriert, bis keine Wasserstoffaufnahme mehr zu beobachten war.

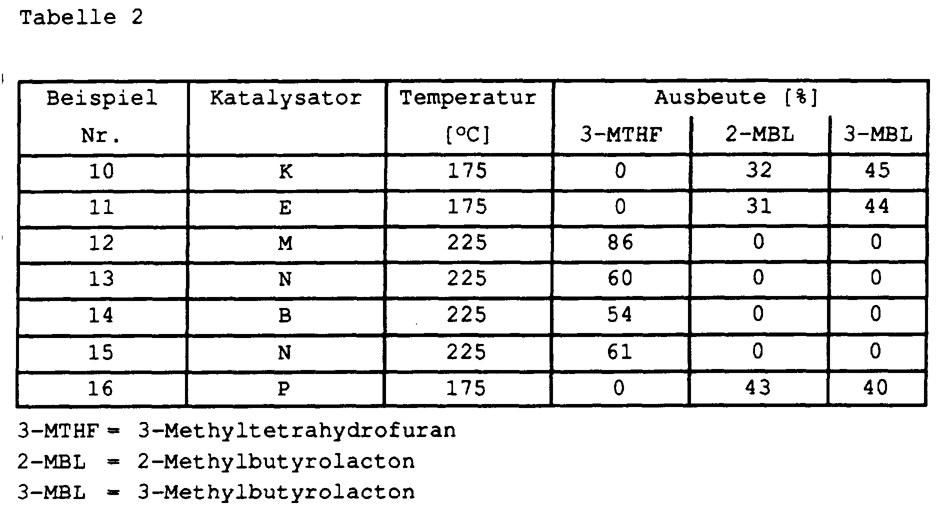

## Patentansprüche

1. Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/oder 3-Methyl-γ-butyrolacton, dadurch gekennzeichnet, daß man Zitronensäure oder deren C₁- bis C₂₀-Alkyl- oder C₇- bis C₁₂-Aralkylester in nicht-wäßrigen Lösungsmitteln bei Temperaturen von 50 bis 400°C und Drücken von 1 bis 400 bar an Hydrierkatalysatoren umsetzt.

2. Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/oder 3-Methyl-γ-butyrolacton nach Anspruch 1, dadurch gekennzeichnet, daß man Hydrierkatalysatoren einsetzt, deren katalytisch aktive Masse eines oder mehrere Elemente der I., VII. oder VIII. Nebengruppe des Periodischen Systems der Elemente enthalten.

3. Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/oder 3-Methyl-γ-butyrolacton nach Anspruch 1, dadurch gekennzeichnet, daß man Hydrierkatalysatoren einsetzt, deren katalytisch aktive Masse eines oder mehrere Elemente der I., VII. oder VIII. Nebengruppe des Periodischen Systems der Elemente und eines oder mehrere Elemente der II. bis VI. Nebengruppe des Periodischen Systems der Elemente enthalten.

4. Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/oder 3-Methyl-γ-butyrolacton nach Anspruch 1, dadurch gekennzeichnet, daß man Hydrierkatalysatoren einsetzt, die Kupfer, Kobalt, Palladium, Nickel und/oder Ruthenium enthalten.

5. Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/oder 3-Methyl-γ-butyrolacton nach Anspruch 1, dadurch gekennzeichnet, daß man Hydrierkatalysatoren einsetzt, die Kupfer und/oder eines oder mehrere Elemente der VIII. Nebengruppe und Molybdän, Wolfram, Mangan, und/oder Zink enthalten.

6. Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/oder 3-Methyl-γ-butyrolacton nach Anspruch 1, dadurch gekennzeichnet, daß man die katalytische Hydrierung bei Temperaturen von 150 bis 300°C und Drücken von 50 bis 300 bar durchführt.

7. Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/oder 3-Methyl-γ-butyrolacton nach Anspruch 1, dadurch gekennzeichnet, daß man als Derivate der Zitronensäure Mono-, Di- und/oder Triester verwendet.

8. Verfahren zur Herstellung von Propan-1,2,3-tricarbonsäure, Tetrahydrofurylessigsäure und deren C₁- bis C₂₀-Alkyl- oder C₇- bis C₁₂-Aralkylester, Propan-1,2,3-trimethanol, 3-Methyltetrahydrofuran, 3-(2'-Hydroxyethyl)tetrahydrofuran, 4-Hydroxymethyltetrahydropyran, 2-Methyl-γ-butyrolacton und/ oder 3-Methyl-γ-butyrolacton nach Anspruch 1, dadurch gekennzeichnet, daß man als nicht-wäßrige Lösungsmittel Alkohole oder Ether verwendet.

## Claims

1. A process for the preparation of propane-1,2,3-tricarboxylic acid, tetrahydrofurfurylacetic acid and the C₁- to C₂₀-alkyl or C₇- to C₁₂-aralkyl esters thereof, propane-1,2,3-trimethanol, 3-methyltetrahydrofuran, 3-(2'-hydroxyethyl)tetrahydrofuran, 4-hydroxymethyltetrahydropyran, 2-methyl-γ-butyrolactone and/or 3-methyl-γ-butyrolactone, which comprises reacting citric acid or the C₁- to C₂₀-alkyl or C₇- to C₁₂-aralkyl esters thereof on hydrogenation catalysts in non-aqueous solvents at from 50 to 400°C and at from 1 to 400 bar.

2. A process as claimed in claim 1 for the preparation of propane-1,2,3-tricarboxylic acid, tetrahydrofurfurylacetic acid and the C₁- to C₂₀-alkyl or C₇- to C₁₂-aralkyl esters thereof, propane-1,2,3-trimethanol, 3-methyltetrahydrofuran, 3-(2'-hydroxyethyl)tetrahydrofuran, 4-hydroxymethyltetrahydropyran, 2-methyl-γ-butyrolactone and/or 3-methyl-γ-butyrolactone, wherein hydrogenation catalysts are employed whose catalytically active material contains one or more elements from sub-group I, VII or VIII of the Periodic Table of the Elements.

3. A process as claimed in claim 1 for the preparation of propane-1,2,3-tricarboxylic acid, tetrahydrofurfurylacetic acid and the C₁- to C₂₀-alkyl or C₇- to C₁₂-aralkyl esters thereof, propane-1,2,3-trimethanol, 3-methyltetrahydrofuran, 3-(2'-hydroxyethyl)tetrahydrofuran, 4-hydroxymethyltetrahydropyran, 2-methyl-γ-butyrolactone and/or 3-methyl-γ-butyrolactone, wherein hydrogenation catalysts are employed whose catalytically active material contains one or more elements from sub-group I, VII or VIII of the Periodic Table of the Elements and one or more elements from sub-groups II to VI of the Periodic Table of the Elements.

4. A process as claimed in claim 1 for the preparation of propane-1,2,3-tricarboxylic acid, tetrahydrofurfurylacetic acid and the C₁- to C₂₀-alkyl or C₇- to C₁₂-aralkyl esters thereof, propane-1,2,3-trimethanol, 3-methyltetrahydrofuran, 3-(2'-hydroxyethyl)tetrahydrofuran, 4-hydroxymethyltetrahydropyran, 2-methyl-γ-butyrolactone and/or 3-methyl-γ-butyrolactone, wherein hydrogenation catalysts are employed which contain copper, cobalt, palladium, nickel and/or ruthenium.

5. A process as claimed in claim 1 for the preparation of propane-1,2,3-tricarboxylic acid, tetrahydrofurfurylacetic acid and the C₁- to C₂₀-alkyl or C₇- to C₁₂-aralkyl esters thereof, propane-1,2,3-trimethanol, 3-methyltetrahydrofuran, 3-(2'-hydroxyethyl)tetrahydrofuran, 4-hydroxymethyltetrahydropyran, 2-methyl-γ-butyrolactone and/or 3-methyl-γ-butyrolactone, wherein hydrogenation catalysts are employed which contain copper and/or one or more elements from sub-group VIII and molybdenum, tungsten, manganese and/or zinc.

6. A process as claimed in claim 1 for the preparation of propane-1,2,3-tricarboxylic acid, tetrahydrofurfurylacetic acid and the C₁- to C₂₀-alkyl or C₇- to C₁₂-aralkyl esters thereof, propane-1,2,3-trimethanol, 3-methyltetrahydrofuran, 3-(2'-hydroxyethyl)tetrahydrofuran, 4-hydroxymethyltetrahydropyran, 2-methyl-γ-butyrolactone and/or 3-methyl-γ-butyrolactone, wherein the catalytic hydrogenation is carried out at from 150 to 300°C and at from 50 to 300 bar.

7. A process as claimed in claim 1 for the preparation of propane-1,2,3-tricarboxylic acid, tetrahydrofurfurylacetic acid and the C₁- to C₂₀-alkyl or C₇- to C₁₂-aralkyl esters thereof, propane-1,2,3-trimethanol, 3-methyltetrahydrofuran, 3-(2'-hydroxyethyl)tetrahydrofuran, 4-hydroxymethyltetrahydropyran, 2-methyl-γ-butyrolactone and/or 3-methyl-γ-butyrolactone, wherein the derivatives of citric acid are monoesters, diesters and/or triesters.

8. A process as claimed in claim 1 for the preparation of propane-1,2,3-tricarboxylic acid, tetrahydrofurfurylacetic acid and the C₁- to C₂₀-alkyl or C₇- to C₁₂-aralkyl esters thereof, propane-1,2,3-trimethanol, 3-methyltetrahydrofuran, 3-(2'-hydroxyethyl)tetrahydrofuran, 4-hydroxymethyltetrahydropyran, 2-methyl-γ-butyrolactone and/or 3-methyl-γ-butyrolactone, wherein the non-aqueous solvents used are alcohols or ethers.

## Revendications

1. Procédé de préparation de l'acide propane-1,2,3-tricarboxylique, de l'acide tétrahydrofurfurylacétique et de leurs esters alkyliques en C₁ à C₂₀ ou de leurs esters aralkyliques en C₇ à C₁₂, du propane-1,2,3-triméthanol, du 3-méthyltétrahydrofuranne, du 3-(2'-hydroxyéthyl) tétrahydrofuranne, du 4-hydroxyméthyltétrahydropyranne, de la 2-méthyl-γ-butyrolactone et/ou de la 3-méthyl-γ-butyrolactone, caractérisé en ce que l'on fait réagir l'acide citrique ou ses esters alkyliques en C₁ à C₂₀, ou ses esters aralkyliques en C₇ à C₁₂ à des températures de 50 à 400°C et sous des pressions de 1 à 400 bars dans des solvants non aqueux sur des catalyseurs d'hydrogénation.

2. Procédé de préparation de l'acide propane-1,2,3-tricarboxylique, de l'acide tétrahydrofurfurylacétique et de leurs esters alkyliques en C₁ à C₂₀ ou de leurs esters aralkyliques en C₇ à C₁₂, du propane-1,2,3-triméthanol, du 3-méthyltétrahydrofuranne, du 3-(2'-hydroxyéthyl)tétrahydrofuranne, du 4-hydroxyméthyltétrahydropyranne, de la 2-méthyl-γ-butyrolactone et/ou de la 3-méthyl-γ-butyrolactone, suivant la revendication 1, caractérisé en ce que l'on utilise des catalyseurs d'hydrogénation dont les masses catalytiquement actives contiennent un ou plusieurs éléments des premier, septième ou huitième sous-groupes du système périodique des éléments.

3. Procédé de préparation de l'acide propane-1,2,3-tricarboxylique, de l'acide tétrahydrofurfurylacétique et de leurs esters alkyliques en C₁ à C₂₀ ou de leurs esters aralkyliques en C₇ à C₁₂, du propane-1,2,3-triméthanol, du 3-méthyltétrahydrofuranne, du 3-(2'-hydroxyéthyl)tétrahydrofuranne, du 4-hydroxyméthyltétrahydropyranne, de la 2-méthyl-γ-butyrolactone et/ou de la 3-méthyl-γ-butyrolactone, suivant la revendication 1, caractérisé en ce que l'on utilise des catalyseurs d'hydrogénation dont les masses catalytiquement actives contiennent un ou plusieurs éléments des premier, septième ou huitième sous-groupes du système périodique des éléments et un ou plusieurs éléments des deuxième à sixième sous-groupes du système périodique des éléments.

4. Procédé de préparation de l'acide propane-1,2,3-tricarboxylique, de l'acide tétrahydrofurfurylacétique et de leurs esters alkyliques en C₁ à C₂₀ ou de leurs esters aralkyliques en C₇ à C₁₂, du propane-1,2,3-triméthanol, du 3-méthyltétrahydrofuranne, du 3-(2'-hydroxyéthyl)tétrahydrofuranne, du 4-hydroxyméthyltétrahydropyranne, de la 2-méthyl-γ-butyrolactone et/ou de la 3-méthyl-γ-butyrolactone, suivant la revendication 1, caractérisé en ce que l'on utilise des catalyseurs d'hydrogénation qui contiennent du cuivre, du cobalt, du palladium, du nickel et/ou du ruthénium.

5. Procédé de préparation de l'acide propane-1,2,3-tricarboxylique, de l'acide tétrahydrofurfurylacétique et de leurs esters alkyliques en C₁ à C₂₀ ou de leurs esters aralkyliques en C₇ à C₁₂, du propane-1,2,3-triméthanol, du 3-méthyltétrahydrofuranne, du 3-(2'-hydroxyéthyl)tétrahydrofuranne, du 4-hydroxyméthyltétrahydropyranne, de la 2-méthyl-γ-butyrolactone et/ou de la 3-méthyl-γ-butyrolactone, suivant la revendication 1, caractérisé en ce que l'on utilise des catalyseurs d'hydrogénation qui contiennent du cuivre et un ou plusieurs éléments du huitième sous-groupe et du molybdène, du tungstène, du manganèse et/ou du zinc.

6. Procédé de préparation de l'acide propane-1,2,3-tricarboxylique, de l'acide tétrahydrofurfurylacétique et de leurs esters alkyliques en C₁ à C₂₀ ou de leurs esters aralkyliques en C₇ à C₁₂, du propane-1,2,3-triméthanol, du 3-méthyltétrahydrofuranne, du 3-(2'-hydroxyéthyl)tétrahydrofuranne, du 4-hydroxyméthyltétrahydropyranne, de la 2-méthyl-γ-butyrolactone et/ou de la 3-méthyl-γ-butyrolactone, suivant la revendication 1, caractérisé en ce que l'on entreprend l'hydrogénation catalytique à des températures de 150 à 300°C et sous des pressions de 50 à 300 bars.

7. Procédé de préparation de l'acide propane-1,2,3-tricarboxylique, de l'acide tétrahydrofurfurylacétique et de leurs esters alkyliques en C₁ à C₂₀ ou de leurs esters aralkyliques en C₇ à C₁₂, du propane-1,2,3-triméthanol, du 3-méthyltétrahydrofuranne, du 3-(2'-hydroxyéthyl)tétrahydrofuranne, du 4-hydroxyméthyltétrahydropyranne, de la 2-méthyl-γ-butyrolactone et/ou de la 3-méthyl-γ-butyrolactone, suivant la revendication 1, caractérisé en ce que l'on utilise, à titre de dérivés de l'acide citrique, les mono-, di- et/ou triesters.

8. Procédé de préparation de l'acide propane-1,2,3-tricarboxylique, de l'acide tétrahydrofurfurylacétique et de leurs esters alkyliques en C₁ à C₂₀ ou de leurs esters aralkyliques en C₇ à C₁₂, du propane-1,2,3-triméthanol, du 3-méthyltétrahydrofuranne, du 3-(2'-hydroxyéthyl)tétrahydrofuranne, du 4-hydroxyméthyltétrahydropyranne, de la 2-méthyl-γ-butyrolactone et/ou de la 3méthyl-γ-butyrolactone, suivant la revendication 1, caractérisé en ce que l'on utilise des alcools ou des éthers à titre de solvants non aqueux.
